# EUROPÄISCHE PATENTANMELDUNG

(11) **EP 0 707 828 A1**
(43) Veröffentlichungstag der Anmeldung: **24.04.1996**
(21) Anmeldenummer: 95103081.6
(22) Anmeldetag: 03.03.1995
(51) Int. Cl.: A61B 6/03, A61B 6/14

(54) **Röntgenaufnahmegerät**

(30) Priorität: 17.10.1994 DE 4437077; 25.11.1994 DE 4441974; 28.12.1994 DE 4446960
(71) Anmelder: Kovacs, Sandor, c/o ISR Computersystem GmbH, D-81377 München (DE)
(72) Erfinder: Kovacs, Sandor, c/o ISR Computersystem GmbH, D-81377 München (DE)
(74) Vertreter: Kern, Ralf M., Dipl.-Ing.

(57) **Zusammenfassung**

Die Erfindung betrifft ein Röntgenaufnahmegerät mit einer Röntgenquelle und einer die durch das Aufnahmeobjekt hindurchgeschickten Röntgenstrahlen zur digitalen Weiterverarbeitung erfassenden Abbildungseinrichtung, wobei die Abbildungseinrichtung aus einer Röntgenwandlerfolie und einem Sensor oder aus einer Speicherfolie und einem Scanner bestehen kann. Solche Röntgenaufnahmegeräte zur digitalen Auswertung von Röntgenaufnahmen können für Kieferpanoramaaufnahmen, Thoraxaufnahmen und Aufnahmen weiterer Körperteile verwendet werden. Bei der Ausführungsform, bei der Röntgenwandlerfolie und Sensor verwendet werden, treffen die Röntgenstrahlen auf die Röntgenwandlerfolie (13), die sich in sichtbares Licht umwandelt. Das Licht wird von einer optischen Linseneinheit (18) auf den Sensor (14) geleitet, der von der Signalaufbereitungseinheit (15a) gesteuert und ausgelesen wird. Die Signalaufbereitungseinheit (15a) separiert, filtert und verstärkt die Bildsignale und führt sie weiter zur Bildspeicherkarte (16). Die Bildspeicherkarte (16) ist mit dem PC (17) verbunden, auf dessen Bildschirm die Röntgenaufnahme dargestellt werden kann und von dem sie archiviert wird.

## Beschreibung

Die Erfindung betrifft ein Röntgenaufnahmegerät mit einer Röntgenquelle und einer Abbildungseinrichtung, wobei insbesondere die von der Abbildungseinrichtung erfaßte Röntgenstrahlung ohne Zuhilfenahme eines röntgenempfindlichen Films digital weiterverarbeitet wird.

Ein solches Röntgenaufnahmegerät ist bereits aus der EP 0 279 294 bekannt. Bei dem bekannten Röntgenaufnahmegerät handelt es sich um eine zahnärztliche Röntgendiagnostikeinrichtung zur Erstellung von Panorama-Schichtaufnahmen vom Kiefer eines Patienten. Hierbei ist eine schwenkbare Einheit vorgesehen, die einen waagrechten Teil und zwei senkrechte Teile aufweist, wobei der waagrechte Teil über dem Kopf des Patienten angeordnet ist und die beiden senkrechten Teile diametral gegenüberliegend jeweils auf einer Seite des Kopfes angeordnet sind. In einem senkrechten Teil ist eine Röntgenquelle untergebracht, die sich in einem Gehäuse befindet, das auf der dem Kopf zugewandten Seite einen senkrechten Schlitz aufweist. Im anderen senkrechten Teil ist die Abbildungseinrichtung untergebracht. Die Röntgenstrahlen, die durch den Kopf gegangen sind, treffen hier auf eine Szintillatorschicht und werden in sichtbares Licht umgewandelt. Das sichtbare Licht wird von einer Glasfaseroptik auf eine Detektoranordnung geführt, die elektrische Signale proportional zur Strahlungsintensität bildet. An die Detektoranordnung schließen sich ein Analog/Digital-Wandler, ein Bildspeicher sowie eine Datenverarbeitungseinrichtung mit Rechner an, der aus den von der Detektoranordnung während eines Aufnahmeablaufs gelieferten Signalen ein Übersichtsbild berechnet.

Bei dem bekannten Röntgenaufnahmegerät ist jedoch die Glasfaseroptik verhältnismäßig aufwendig und macht das Gerät daher relativ teuer.

Eine Aufgabe der Erfindung ist es daher, ein Röntgenaufnahmegerät mit den Merkmalen des Oberbegriffs von Anspruch 1 bereitzustellen, das diesen Nachteil nicht aufweist.

Eine weitere Aufgabe der Erfindung ist es, ein Röntgenaufnahmegerät mit den Merkmalen des Oberbegriffs des Anspruchs 5 bereitzustellen, bei dem defekte und daher besonders billige Sensoren Anwendung finden können.

Eine dritte Aufgabe der Erfindung ist es, ein Röntgenaufnahmegerät mit den Merkmalen des Oberbegriffs von Anspruch 29 bereitzustellen, bei der die Auswertung der Röntgenaufnahme außerhalb des Röntgenaufnahmegeräts erfolgen kann, das deshalb besonders kleine räumliche Abmessungen haben kann.

Die erste Aufgabe wird für ein Röntgenaufnahmegerät mit den Merkmalen des Oberbegriffs von Anspruch 1 durch das Merkmal des kennzeichnenden Teils von Anspruch 1 gelöst. Die dabei verwendete Linsenoptik ist wesentlich einfacher und preiswerter als die im Stand der Technik verwendete Glasfaseroptik.

Die zweite Aufgabe wird für ein Röntgenaufnahmegerät mit den Merkmalen des Oberbegriffs von Anspruch 5 durch die Merkmale des kennzeichnenden Teils von Anspruch 5 gelöst. Dadurch, daß mehrere Sensoren in einer Reihe angeordnet sind, braucht bloß ein Bruchteil der Sensorfläche des einzelnen Sensors zu funktionieren, so daß bei den Sensoren billige Ausschußware verwendet werden kann, was große Vorteile bietet. Die einzelnen Glasfaseroptiken für die Sensoren sind ebenfalls preisgünstiger als eine einzige größere Glasfaseroptik für das Gesamtsystem.

Die dritte Aufgabe wird für eine Röntgenaufnahmegerät mit den Merkmalen des Oberbegriffs von Anspruch 29 durch die Merkmale des kennzeichnenden Teils von Anspruch 29 gelöst. Die verwendete Speicherfolie kann nach Bestrahlung aus dem Röntgenaufnahmegerät herausgenommen werden und außerhalb von dem Scanner abgelesen werden. Dadurch kann das Röntgenaufnahmegerät sehr klein gemacht werden.

Weitere Ausgestaltungen der Erfindung sind den Unteransprüchen zu entnehmen.

Ein besonders bevorzugtes Ausführungsbeispiel ist anhand der Figuren erläutert, in denen:
Fig. 1 die Seitenansicht eines Röntgenaufnahmegeräts zur Erstellung von Kieferpanoramaaufnahmen mit Patientenkopf zeigt;
Fig. 2 ein Blockschaltbild ist, das eine Ausführungsform des erfindungsgemäßen Röntgenaufnahmegeräts zeigt;
Fig. 3 Röntgenwandlerfolie, optische Glasfasereinheiten und Sensoren bei einer Ausführungsform zeigt, bei der mehrere aufgereihte Sensoren verwendet werden;
Fig. 4 Röntgenwandlerfolie, konvexe Linse und Sensor bei einer weiteren Ausführungsform zeigt,
Fig. 5 Röntgenwandlerfolie, Zylinderlinse und Sensor bei einer weiteren Ausführungsform zeigt, und
Fig. 6 Röntgenwandlerfolie, Spiegel, kovexe Linse und Sensor bei einer weiteren Ausführungsform zeigt.

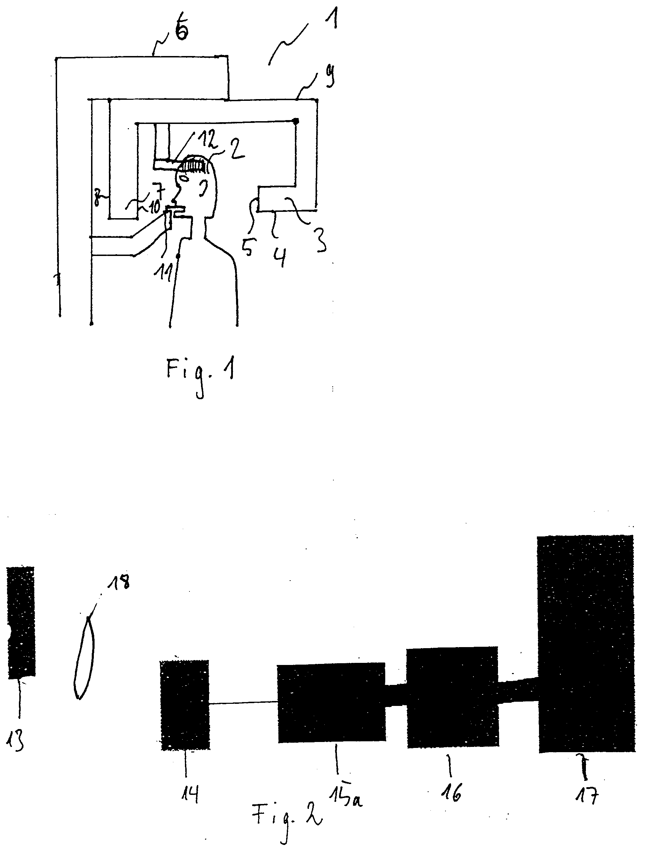

In Fig. 1 ist ein Röntgenaufnahmegerät für Kieferpanoramaaufnahmen zu erkennen, wie es, mit einem Röntgenfilm ausgerüstet, in vielen Zahnarztpraxen Verwendung findet. Im Fall der vorliegenden Erfindung ist es statt mit einem Röntgenfilm entweder mit einem Sensor und einer Röntgenwandlerfolie, oder mit einer Speicherfolie und einem Scanner ausgerüstet. An einem Traggerüst 6 ist eine Einheit 9 schwenkbar befestigt. Die Einheit 9 hat einen waagerechten Teil und zwei senkrechte Teile, zwischen denen sich der Kopf des Patienten befindet. In dem einen senkrechten Teil befindet sich in Höhe des Kopfs 2 ein Gehäuse 4, in dem sich die Röntgenquelle 3 befindet, die bei der erfindungsgemäßen Ausführung ihre Strahlen durch einen senkrechten Spalt 5 schickt, der an der dem Kopf 2 zugewandten Seite des Gehäuses 4 angeordnet ist. An dem anderen senkrechten Teil der Einheit 9 befindet sich in Kopfhöhe das Gehäuse 8 der Abbildungseinrichtung 7, wobei die durch den Kopf hindurchgeschickten Strahlen durch einen ebenfalls senkrechten Spalt im Gehäuse 8 auf die Abbildungseinrichtung 7 treffen. Die Einheit 9 wird langsam um den Kopf 2 des Patienten herumgeschwenkt, wobei sich dieser Kopf 2 immer auf der Verbindungslinie zwischen der Röntgenquelle und der Abbildungseinrichtung befindet. Auf diese Weise entsteht nach und nach eine Panoramaaufnahme des Kiefers. Zur besseren Justierung des Kopfs 2 des Patienten sind ein Aufbißblock 11, der sich auf einer am Traggerüst 6 befindlichen Aufbißblockkonsole 18 befindet, und eine ebenfalls am Traggerüst 6 befestigte Nasen-Stirn-Stütze 12 vorgesehen.

Erfindungsgemäß können statt des einen senkrechten Spaltes 5 zwei parallele Spalte 5 vorgesehen sein, die von einer Abdeckeinrichtung abwechselnd so verdeckt werden, daß jeweils ein Spalt 5 frei ist, und somit nacheinander zwei Röntgenaufnahmen entstehen, die auf eine später zu beschreibende Weise zu einer Stereoaufnahme vereinigt werden. Es ist nach einer besonderen Ausführungsform der Erfindung auch möglich, über den Spalt 5 einen beweglichen Querspalt anzuordnen, der sich bezüglich der Schwenkgeschwindigkeit sehr schnell bewegt, so daß eine Abbildung des Spalts 5 entsteht, die sich aus verschiedenen Abtastelementen zusammensetzt.

Fig. 2 zeigt eine besonders bevorzugte Ausführungsform der Erfindung in einem Blockschaltbild. Die Röntgenstrahlen, z.B. eines Röntgenaufnahmegerätes für Kieferpanoramaaufnahmen, treffen von links auf die Röntgenwandlerfolie 13, die sie in sichtbares Licht verwandelt. Das sichtbare Licht wird anschließend über eine optische Linseneinheit 18 auf einen Sensor 14 geleitet, der ein Bild aufnimmt. Bei dem Sensor 14 kann es sich um einen Zeilensensor oder um einen Flächensensor mit in der Fläche verteilten Bildpunkten handeln. Bei dem Zeilensensor kann es sich um ein CCD (Charge Coupling Device) oder um einen spektroskopischen Hochleistungsbildsensor handeln. Der Sensor 14 wird durch die Steuereinheit 15a angesteuert und ausgelesen, wobei die Ansteuerung bei einem Zeilensensor im Inverted Mode erfolgt. Die spezielle Bauweise des spektroskopischen Hochleistungsbildsensors erlaubt eine hohe Lichtkonstante durch Aufaddierung der Lichtsignale in einem Schieberegister. Der zum Auslesen des Zeilensensors erforderliche Zeittakt wird von der Steuereinheit 15a vorgegeben. Die Steuereinheit 15a, die auch als Signalaufbereitungseinheit bezeichnet wird, separiert, filtert und verstärkt die ankommenden Signale. Die Steuereinheit 15a ist zum Filtern und Verstärken mit einer Filter- und einer Verstärkereinheit ausgestattet. Die verwertbaren Informationen werden dann durch ein internes Bussystem zu einer Bildspeicherkarte 16 geführt, die mit einem Personal Computer (PC) 17 verbunden ist. In dem PC 17 können die Bildsignale zusammengeführt und mit einer speziellen Software auf dem Bildschirm dargestellt werden. Die Signale reichen aus, um eine Diagnose wie bei einer Filmaufnahme zu ermöglichen. Der PC 17 ist an ein Informationssystem angebunden. In diesem Informationssystem werden die Bilder zu Dokumentationszwecken in einer elektronischen Kartei auf z.B. in einer Optospeichereinheit zum Zahnarztabrechnungssystem abgelegt und können jederzeit ausgedruckt werden. Bei der Ausführungsform mit den zwei parallelen Spalten 5, die wechselweise von der Abdeckungseinheit verdeckt werden, erzeugt das durch das Blockschaltbild 2 vorgegebene Auswertungssystem auf dem Bildschirm des PC 17 zwei verschiedenfarbige Bilder, die gegeneinander verschoben sind. Wenn der Beobachter eine Brille trägt, deren beide Gläser jeweils in der betreffenden Farbe gefärbt sind, kann er eine Stereoröntgenaufnahme erblicken.

Bei der Ausführungsform, bei der ein beweglicher Querschlitz über den Spalt 5 hinwegbewegt wird, setzt sich das auf dem Bildschirm des PCs 17 dargestellte Bild aus den abgetasteten Bereichen der jeweiligen Spaltaufnahme zusammen, wobei die durch die langsame Schwenkbewegung entstehenden Spaltaufnahmen wiederum zu einem Gesamtbild zusammengefügt werden.

Erfindungsgemäß sind nicht nur Röntgenaufnahmegeräte für Kieferpanoramaaufnahmen, wie in Fig. 1 dargestellt, möglich, sondern Röntgenaufnahmegeräte für alle möglichen Körperteile wie z.B. Thorax- oder Wirbelsäulenaufnahmen.

Es ist auch möglich, das Röntgenaufnahmegerät als Computertomograph auszulegen.

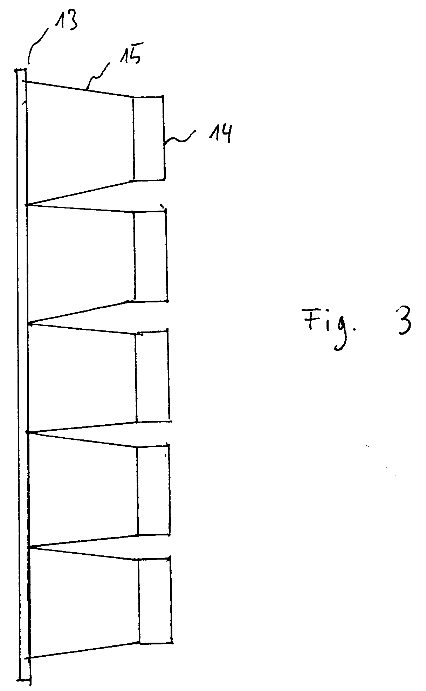

In Fig. 3 ist eine Ausführungsform zu erkennen, bei der fünf senkrecht aufgereihte Sensoren 14 verwendet werden, auf die das sichtbare Licht von der Röntgenwandlerfolie 13 mit Hilfe von fünf optischen Glasfasereinheiten 15 geleitet wird. Bei dieser Anordnung sind die optischen Glasfasereinheiten 15 an der Röntgenwandlerfolie 13 einander unmittelbar benachbart und laufen prismaförmig auf die Sensoren 14 zu. Bei dieser Ausführungsform bestehen die beiden Vorteile, daß erstens die verwendeten fünf kleinen optischen Glasfasereinheiten 15 insgesamt preiswerter sind, als die größere optische Glasfasereinheit des Stands der Technik und eine größere Sensorkapazität zur Verfügung steht, wodurch es ermöglicht wird, Sensoren zu verwenden, die nicht hundertprozentig funktionieren und daher als Ausschußware sehr preiswert sind. so ist es zum Beispiel bei der Verwendung von Zeilensensoren möglich auf Sensoren zurückzugreifen, bei denen nur ein Fünftel der Zeilen funktioniert.

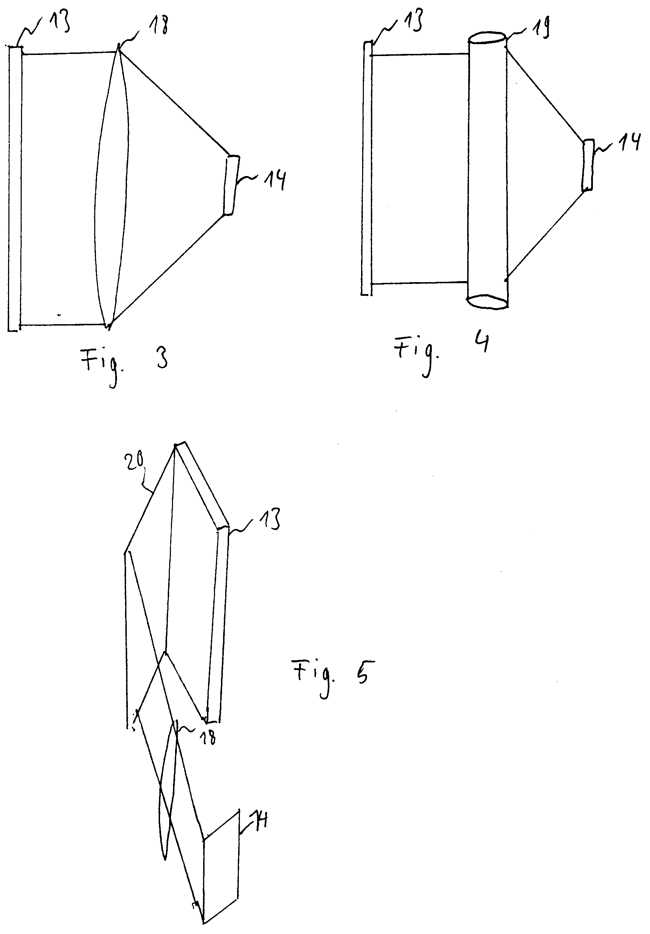

In Fig. 4 ist eine Ausführungsform dargestellt, bei der wiederum nur ein Sensor 14 Verwendung findet. Dafür wird eine konvexe Linse 18 oder ein Linsensystem 18 aus konvexen Linsen verwendet, um das sichtbare Licht von der Röntgenwandlerfolie 13 zum Sensor 14 zu leiten.

Fig. 5 entspricht Fig. 4, mit dem Unterschied, daß in Fig. 5 eine Zylinderlinse 19 oder ein Linsensystem 19 mit Zylinderlinsen verwendet wird.

In Fig. 6 ist der Sensor 14 seitlich von der Röntgenwandlerfolie 13 und in einem bestimmten Winkel zu ihr angeordnet. Hier wird das von der Röntgenwandlerfolie 13 ausgestrahlte Licht durch einen Spiegel 20 umgelenkt, der in einem bestimmten Winkel zur Röntgenwandlerfolie 13 eingeordnet ist, und durch eine konvexe Linse 18 zum Sensor 14 geleitet. Statt des Spiegels 20 kann auch ein Prisma Verwendung finden.

Allgemein kann zwischen Röntgenwandlerfolie 13 und Sensor 14 auch noch ein Lichtverstärker angeordnet werden, um die Empfindlichkeit des Röntgenaufnahmegerätes zu erhöhen.

## Patentansprüche

1. Röntgenaufnahmegerät mit einer von einem strahlungsabsorbierenden Gehäuse (4) mit mindestens einem Spalt (5) auf seiner dem Aufnahmeobjekt (2) zugewandten Seite umgebenen Röntgenquelle (3), einer die Röntgenstrahlen zur digitalen Weiterverarbeitung erfassenden Abbildungseinrichtung (7), die eine Röntgenwandlerfolie (13) zur Umwandlung von Röntgenstrahlen in niederfrequentere Strahlung, einen Sensor (14) oder mehrere Sensoren (14) und eine die niederfrequentere Strahlung von der Röntgenwandlerfolie (13) auf den Sensor (14) oder die Sensoren (14) leitende optische Einheit (18, 19, 20) aufweist, wobei die Röntgenquelle (3) und die Abbildungseinrichtung am Aufnahmeobjekt entlangfahren und dabei nach und nach die Gesamtaufnahme entsteht, einer Signalaufbereitungseinheit (15a), von der der Sensor (14) bzw. die Sensoren (14) gesteuert und ausgelesen wird bzw. werden, einem internen Bussystem, einer Bildspeicherkarte (16), wobei die verwertbaren Signale aus der Signalaufbereitungseinheit (15a) von dem internen Bussystem zu der Bildspeicherkarte (16) geführt werden, und einem PC-System (17) mit Anbindung an ein Informationssystem, wobei die Bildspeicherkarte (16) mit dem PC-System (17) verbunden ist und die Röntgenaufnahme auf dem Bildschirm des PC-Systems (17) dargestellt wird, **dadurch gekennzeichnet,** daß die optische Einheit (18, 19, 20) ein Linsensystem (18, 19) aufweist, das vorzugsweise aus einer Linse (18, 19) besteht.

2. Röntgenaufnahmegerät nach Anspruch 1, **dadurch gekennzeichnet,** daß die Röntgenwandlerfolie (13) und der Sensor (14) bzw. die Sensoren (14) seitlich gegeneinander verschoben sind und die optische Einheit (18, 19, 20) durch ein Prisma oder einen Spiegel (20) zum Umlenken der niederfrequenteren Strahlung erweitert ist.

3. Röntgenaufnahmegerät nach einem der Ansprüche 1 oder 2, **dadurch gekennzeichnet,** daß das Linsensystem (18, 19) aus konvexen Linsen (18) oder einer konvexen Linse (18) besteht.

4. Röntgenaufnahmegerät nach einem der Ansprüche 1 oder 2, **dadurch gekennzeichnet,** daß das Linsensystem (18, 19) aus Zylinderlinsen (19) oder einer Zylinderlinse (19) besteht.

5. Röntgenaufnahmegerät mit einer von einem strahlungsabsorbierenden Gehäuse (4) mit mindestens einem Spalt (5) auf seiner dem Aufnahmeobjekt (2) zugewandten Seite umgebenen Röntgenquelle (3), einer die Röntgenstrahlen zur digitalen Weiterverarbeitung erfassenden Abbildungseinrichtung (7), die eine Röntgenwandlerfolie (13) zur Umwandlung von Röntgenstrahlen in niederfrequentere Strahlung, mehrere Sensoren (14) und eine die niederfrequentere Strahlung von der Röntgenwandlerfolie (13) auf die Sensoren (14) leitende optische Einheit (15) aufweist, wobei die Röntgenquelle (3) und die Abbildungseinrichtung (7) am Aufnahmeobjekt entlangfahren und dabei nach und nach die Gesamtaufnahme entsteht, einer Signalaufbereitungseinheit (15a), von der die Sensoren (14) gesteuert und ausgelesen werden, einem internen Bussystem, einer Bildspeicherkarte (16), wobei die verwertbaren Signale aus der Signalaufbereitungseinheit (15a) von dem internen Bussystem zu der Bildspeicherkarte (16) geführt erden, und einem PC-System (17) mit Anbindung an ein Informationssystem, wobei die Bildspeicherkarte (16) mit dem PC-System (17) verbunden ist und die Röntgenaufnahme auf dem Bildschirm des PC-Systems (17) dargestellt wird, daß die Sensoren (14) parallel zur Richtung des Spalts (5) im Röntgenquellengehäuse (4) aufgereiht sind, und die optische Einheit (15) aus einer Reihe von Glasfasereinheiten (15) besteht, wobei jedem Sensor (14) eine Glasfasereinheit (15) zugeordnet ist.

6. Röntgenaufnahmegerät nach Anspruch 5, **dadurch gekennzeichnet,** daß die Glasfasereinheiten (15) von der Röntgenwandlerfolie (13) einander unmittelbar benachbart sind und prismaförmig auf die Sensoren (14) zulaufen, die in einem gewissen Abstand voneinander angeordnet sind.

7. Röntgenaufnahmegerät nach einem der Ansprüche 1 bis 6, **dadurch gekennzeichnet,** daß in der dem Aufnahmeobjekt (2) zugewandten Seite des Röntgenquellengehäuses (4) zwei parallele Spalte (5) vorgesehen sind, für die beiden parallelen spalte (5) Abdeckvorrichtungen vorgesehen sind, die Abdeckvorrichtungen die Spalte (5) abwechselnd so verdecken, daß jeweils ein Spalt (5) frei ist, und jeweils ein Bild von einem Spalt (5) in einer bestimmten Farbe auf dem Bildschirm erscheint, wobei den beiden Spalten (5) zwei verschiedene Farben entsprechen, so daß von einem Beobachter, der eine Brille mit zwei in den beiden verschiedenen Farben gefärbten Gläsern trägt, eine Röntgenstereoaufnahme beobachtet werden kann.

8. Röntgenaufnahmegerät nach einem der Ansprüche 1 bis 7, **dadurch gekennzeichnet,** daß über dem mindestens einen Spalt (5) oder den Spalten (5) im Gehäuse (4) der Röntgenquelle (3) eine Abdeckung mit einem beweglichen Querspalt angeordnet ist, der sich bezüglich der Geschwindigkeit von Röntgenquelle (3) und Abbildungseinrichtung (7) sehr schnell bewegt, so daß sich die Abbildung auf dem Bildschirm aus den Abbildungen der Spalte (5) oder der Spalte (5) zusammensetzt, die sich aus verschiedenen Abtastelementen zusammensetzen.

9. Röntgenaufnahmegerät nach einem der Ansprüche 1 bis 8, **dadurch gekennzeichnet,** daß es sich bei dem Sensor (14) bzw. den Sensoren (14) um einen Zeilensensor bzw. Zeilensensoren handelt, dessen bzw. deren Zeilen parallel zum Spalt (5) im Röntgenquellengehäuse angeordnet sind.

10. Röntgenaufnahmegerät nach einem der Ansprüche 1 bis 8, **dadurch gekennzeichnet,** daß es sich bei dem Sensor (14) bzw. den Sensoren (14) um einen Flächensensor bzw. Flächensensoren mit auf der Fläche verteilten Bildpunkten handelt.

11. Röntgenaufnahmegerät nach einem der Ansprüche 1 bis 10, **dadurch gekennzeichnet,** daß der Sensor (14) bzw. die Sensoren (14) von einer Signalaufbereitungseinheit (15a) im Inverted Mode gesteuert wird bzw. werden.

12. Röntgenaufnahmegerät nach einem der Ansprüche 1 bis 11, **dadurch gekennzeichnet,** daß die in der Signalaufbereitungseinheit (15a) ankommenden Signale separiert und gefiltert werden.

13. Röntgenaufnahmegerät nach Anspruch 12, **dadurch gekennzeichnet,** daß in der Signalaufbereitungseinheit (15a) eine Verstärkereinheit vorgesehen ist, und die separierten und gefilterten Signale von der Verstärkereinheit verstärkt werden.

14. Röntgenaufnahmegerät nach einem der Ansprüche 1 bis 13, **dadurch gekennzeichnet,** daß die Röntgenaufnahmen vom PC-System (17) auf eine Optospeichereinheit abgespeichert werden.

15. Röntgenaufnahmegerät nach einem der Ansprüche 1 bis 14, **dadurch gekennzeichnet,** daß das Röntgenaufnahmegerät (1) als Computertomograph ausgelegt ist.

16. Röntgenaufnahmegerät nach einem der Ansprüche 1 bis 15, **dadurch gekennzeichnet,** daß zwischen der Röntgenwandlerfolie (13) oder dem Sensor (14) bzw. den Sensoren (14) eine Röntgenstrahlenabschirmfolie zum Schutz des Sensors (14) bzw. der Sensoren (14) angeordnet ist.

17. Röntgenaufnahmegerät nach einem der Ansprüche 1 bis 14 oder 16, **dadurch gekennzeichnet,** daß es sich um ein Röntgenaufnahmegerät (1) zur Erstellung von Kieferpanoramaaufnahmen handelt, das mit einem Traggerüst (6) und einer um einen Kopf (2) herum schwenkbar an dem Traggerüst angebrachten Einheit (9) ausgerüstet ist, wobei in der Einheit (9) auf der einen Seite des Kopfs (2) die Röntgenstrahlabbildungseinheit (7) untergebracht ist und auf der anderen Seite die Röntgenquelle (3).

18. Röntgenaufnahmegerät nach Anspruch 18, **gekennzeichnet** durch einen Aufbißblock (11) und eine Nasen-Stirn-Stütze (12) für den Kopf (2)

19. Röntgenaufnahmegerät nach einem der Ansprüche 17 oder 18, **dadurch gekennzeichnet,** daß der Spalt (5) bzw. die Spalte (5) im Gehäuse (4) der Röntgenquelle (3) senkrecht ist bzw. sind.

20. Röntgenaufnahmegerät nach Anspruch 19, **dadurch gekennzeichnet,** daß in der schwenkbaren Einheit (9) ein Gehäuse (8) für die Röntgenstrahlabbildungseinheit (7) vorgesehen ist, und dieses Gehäuse (8) an der dem Kopf (2) zugewandten Seite einen senkrechten Spalt (10) aufweist.

21. Röntgenaufnahmegerät nach einem der Ansprüche 1 bis 16, **dadurch gekennzeichnet,** daß es sich um ein Röntgenaufnahmegerät (1) zur Erstellung von Thoraxaufnahmen handelt.

22. Röntgenaufnahmegerät nach einem der Ansprüche 1 bis 16, **dadurch gekennzeichnet,** daß es sich um ein Röntgenaufnahmegerät (1) zur Erstellung von Schädel- und Halswirbelsäulenaufnahmen handelt.

23. Röntgenaufnahmegerät nach einem der Ansprüche 1 bis 16, **dadurch gekennzeichnet,** daß es sich um ein Röntgenaufnahmegerät (1) zur Erstellung von Bauch- und Lendenwirbelsäulenaufnahmen handelt.

24. Röntgenaufnahmegerät nach einem der Ansprüche 1 bis 16, dadurch gekennzeichnet, daß es sich um ein Röntgenaufnahmegerät (1) zur Erstellung von Arm- und Handaufnahmen handelt.

25. Röntgenaufnahmegerät nach einem der Ansprüche 1 bis 16, **dadurch gekennzeichnet,** daß es sich um ein Röntgenaufnahmegerät (1) zur Erstellung von Unterleibs- und Beckenaufnahmen handelt.

26. Röntgenaufnahmegerät nach einem der Ansprüche 1 bis 16, **dadurch gekennzeichnet,** daß es sich um ein Röntgenaufnahmegerät (1) zur Erstellung von Bein- und Fußaufnahmen handelt.

27. Röntgenaufnahmegerät nach einem der Ansprüche 1 bis 16, **dadurch gekennzeichnet,** daß es sich um ein Röntgenaufnahmegerät (1) zur Erstellung von Ganzkörperaufnahmen handelt.

28. Röntgenaufnahmegerät nach einem der Ansprüche 1 bis 27, **gekennzeichnet** durch einen zwischen der Röntgenwandlerfolie (13) und der optischen Einheit (15, 18, 19) angeordneten Lichtverstärker.

29. Röntgenaufnahmegerät mit einer von einem strahlungsabsorbierenden Gehäuse (4) mit mindestens einem Spalt (5) auf seiner dem Aufnahmeobjekt (2) zugewandten Seite umgebenen Röntgenquelle und einer die Röntgenstrahlen zur digitalen Weiterverarbeitung erfassenden Abbildungseinrichtung (7), **dadurch gekennzeichnet,** daß die Abbildungseinrichtung (7) eine Speicherfolie und einen Scanner aufweist, wobei die Speicherfolie die auf sie treffende Röntgenstrahlung speichert und die gespeicherte Strahlung vom Scanner abgetastet, gelesen und in ein Bild verwandelt wird.

30. Röntgenaufnahmegerät nach einem der Anspruch 1 bis 28, **dadurch gekennzeichnet**, daß die Röntgenwandlerfolie (13) die Röntgenstrahlung in sichtbares Licht umwandelt.

31. Röntgenaufnahmegerät nach einem der Ansprüche 1 bis 28, **dadurch gekennzeichnet,** daß die Röntgenwandlerfolie (13) die Röntgenstrahlung in ultraviolettes Licht umwandelt.
